# EUROPEAN PATENT SPECIFICATION

(11) **EP 4 104 798 B1**
(45) Date of publication and mention of the grant of the patent: **02.12.2026**
(21) Application number: 22170009.9
(22) Date of filing: 26.04.2022
(51) Int. Cl.: A61F 2/42

(54) **UNICOMPARTMENTAL ANKLE PROSTHESIS**
UNIKOMPARTIMENTELLE KNÖCHELPROTHESE
PROTHÈSE DE CHEVILLE UNICOMPARTIMENTALE

(30) Priority: 15.06.2021 US 202163210621 P; 21.04.2022 US 202217660047
(43) Date of publication of application: 21.12.2022
(73) Proprietor: Wright Medical Technology, Inc., Memphis, TN 38117 (US)
(72) Inventor: STROHKIRCH, Terrance W., Memphis, 38103 (US); MCGINLEY, Shawn E., Arlington, 38002 (US)
(74) Representative: Zimmermann & Partner Patentanwälte mbB

(56) References cited:
- US-A- 4 021 864
- US-A1- 2015 320 567

## Description

### BACKGROUND

### 1. Field of Disclosure

This disclosure relates to prosthetics generally, and more specifically to prostheses for a unicompartmental ankle replacement.

### 2. Description of the Related Art

The ankle is a joint that acts much like a hinge. The joint is formed by the union of three bones. The ankle bone is the talus. The top of the talus fits inside a socket that is formed by the lower end of the tibia, and the fibula, the small bone of the lower leg. Arthritis, bone degeneration, and/or injury can cause ankle joint deterioration resulting in pain, reduced range of motion, and decreased quality of life. In many cases, physicians are recommending ankle replacement surgery with a prosthetic implant as an option. Consequently, improved prostheses for ankle replacements are desirable.

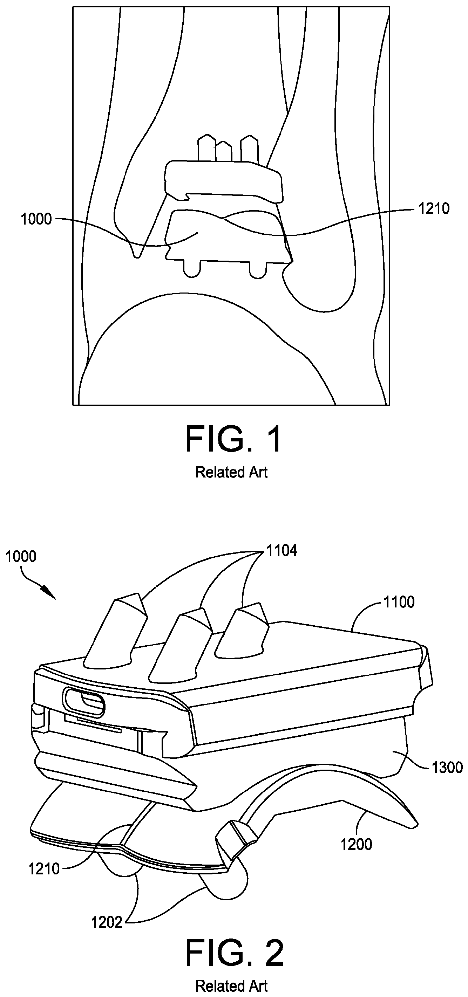

Fig. 1 is a representation of a radiograph of a front of a patient's right ankle including a complete ankle replacement prosthesis 1000 such as the Infinity^{™} Total Ankle Replacement from Wright Medical, according to the related art. Fig. 2 is an isometric view of the ankle replacement prosthesis 1000 shown in Fig. 1. As shown, the ankle replacement prosthesis 1000 includes a tibial implant 1100, a talar implant 1200, and a poly implant 1300. Upon completion of an ankle replacement procedure, the tibial implant 1100 is connected to the tibia, with pegs 1104 located within peg holes in the resected tibia. The talar implant 1200 is connected to the talus, with talar dome anterior pegs 1202 located within holes in the talus. The poly implant 1300 is inserted and located between the tibial implant 1100 and the talar implant 1200.

In such a system, the tibial implant 1100, conventionally made of titanium, covers the bottom (distal end) of the shinbone (tibia) to provide a large footprint for fixation and minimize implant subsidence. The poly implant 1300, conventionally made of polyethylene, acts as the articulating surface of the distal shinbone (tibia) and glides along the contours of the ankle i.e., the talar implant 1200, a highly polished dual metal dome structure. The talar implant 1200 replaces the proximal end (top) of the talus (ankle) bone. The talar implant 1200 acts as the surface upon which the poly implant 1300 glides.

As shown in Figs. 1 and 2, the top of the talar implant 1200 is shaped with two adjacent rounded convex surfaces that are separated by a sulcus or groove 1210. This construction allows for full freedom of movement in the patient 's ankle joint while providing the needed dynamic stabilization.

Document US 4,021,864 relates to a partial prosthesis for a human ankle joint has a metal tibial member with a flat triangular stem, which is implanted in the tibia, wherein the base of the tibial member has a hollow toroidal section in its bottom, wherein the talar member has an upper toroidal section which is engaged by that in the tibial member to provide articulation in the lateral/medial plane and in the anterior/posterior plane with inherent torsional stability.

US 2015/0320567 A1 relates to a total ankle replacement prosthesis may comprise a tibial implant, a talar implant, and an intermediate implant, wherein the intermediate implant may fixedly attach to the tibial implant and may articulate with respect to the talar implant, wherein the intermediate implant may have unequal front and back angular extent, so as to discourage a particular direction of subluxation, and a kit may be provided containing various such intermediate implants, wherein various features may be provided in regard to dovetails, fins, recesses, the placement of fins and pegs, and the shape of the perimeter of the tibial implant, and wherein recesses may allow a surgical blade to slice a latch off of an already-installed intermediate implant, in order to allow its removal from the tibial implant..

This total ankle system is intended to give a patient limited mobility by reducing pain, restoring alignment, and replacing the flexion and extension movement in the ankle joint and is indicated for patients with ankle joints damaged by severe rheumatoid, posttraumatic, or degenerative arthritis. However, this total ankle system does not address the need for a partial replacement of the ankle joint where either only the medial or lateral side is degenerative or has a defect.

### SUMMARY OF THE INVENTION

The invention is defined in claim 1. To overcome the problems described above, preferred embodiments of the present disclosure provide a first step or bridge to conventional treatment and a total ankle in a partially degenerative ankle or an ankle with a unicompartment defect. A unicompartmental ankle prosthesis of the present invention covers either the medial or lateral side of an ankle preserving more bone and being less invasive than a total ankle replacement.

A partial ankle prosthesis includes a tibial component including a superior surface configured to be attached to a lower end of a patient's tibia; a poly component that is fixedly attached to the tibial component and includes a concave inferior surface of an anti-friction material; and a talar component including an inferior surface configured to be attached to a patient's talus and a single convex superior surface configured to mate with the inferior surface of the poly component; and the partial ankle prosthesis is configured to be implanted in a single compartment of a patient's ankle.

The superior surface of the tibial component can includes an outwardly projecting peg configured to fit into a hole in the patient's tibia. The anterior surface of the talar component can include a peg configured to fit into a hole in the patient's talus.

In an embodiment, the superior surface of the talar component can have only one continuous concave curvature both longitudinally between opposite anterior and posterior ends and laterally between opposite medial and lateral sides. The anterior surface of the talar component may advantageously define one or more grooves so as to provide additional surface area and thereby increase bone adhesion. The talar component may also define one or more a notches arranged on the prosthesis so as to interface with a surgical assembly tool.

The partial ankle prosthesis can further include a bioactive coating on a surface of the tibial component and/or the talar component that interfaces with a patient's bone.

An inferior surface of the talar component can be curved and configured to attach to a talar dome of the talus. The inferior surface of the talar component can be substantially flat.

Further disclosed herein is a partial ankle prosthesis that includes a tibial component including a superior surface configured to be attached to a lower end of a patient's tibia; a poly component that is fixedly attached to the tibial component and includes a concave inferior surface of an anti-friction material; and a talar component including an inferior surface configured to be attached to a patient's talus and a single convex superior surface configured to mate with the inferior surface of the poly component; and the partial ankle prosthesis is configured to be implanted in a single compartment of a patient's ankle.

The above and other features, elements, characteristics, steps, and advantages of the present invention will become more apparent from the following detailed description of preferred embodiments of the present invention with reference to the attached drawings.

### BRIEF DESCRIPTION OF THE DRAWINGS

The description of the exemplary embodiments disclosed herein are intended to be read in connection with the accompanying drawings, which are to be considered part of the entire written description. All drawing figures are schematic illustrations and are not intended to show actual dimensions or proportions.
Fig. 1 is a representation of a radiograph of an ankle including an ankle replacement prosthesis according to the related art.
Fig. 2 is an isometric view of an ankle replacement prosthesis in accordance with the related art.
Fig. 3 is an image showing two embodiments of the present disclosure.
Fig. 4 is an isometric view representing a lateral unicompartmental ankle prosthesis in an ankle.
Fig. 5 is a side view representing a lateral unicompartmental ankle prosthesis in an ankle.
Fig. 6 is a bottom isometric view of a three-piece lateral unicompartmental ankle prosthesis.
Fig. 7 is an exploded view of the lateral unicompartmental ankle prosthesis of Fig. 6.
Fig. 8 is an underside view of a tibial component.
Fig. 9 is an underside view of a poly component.
Fig. 10 is a top side view of a poly component.
Fig. 11 is an underside view of the talar component 640 shown without the bioactive coating 670 and including grooves 668 and notch 667.
Figs. 12 and 13 show another embodiment of a medial unicompartmental ankle prosthesis.

### DETAILED DESCRIPTION OF PREFERRED EMBODIMENTS

This description of the exemplary embodiments is intended to be read in connection with the accompanying drawings, which are to be considered part of the entire written description. The drawing figures are not necessarily to scale, and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," "extending" projecting" etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures.

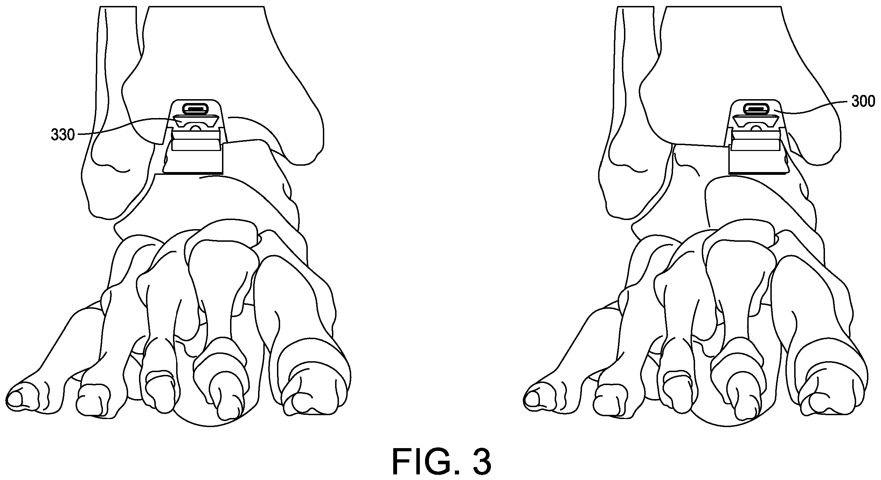

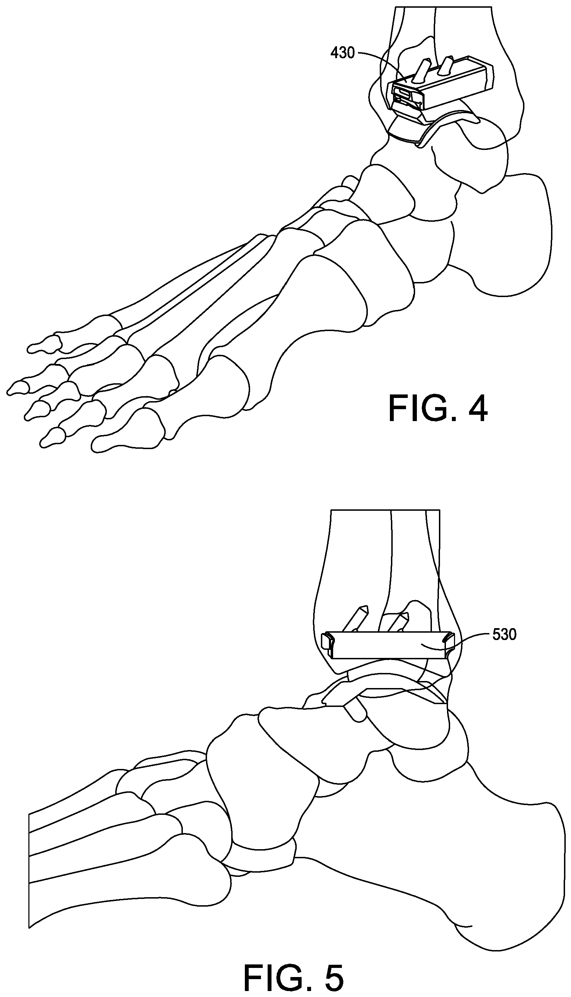

Fig. 3 is an image showing two embodiments of the present disclosure. In Fig. 3, two side-by-side skeletal images of a patient's right foot represent a medial unicompartmental ankle prosthesis 300 and a lateral unicompartmental ankle prosthesis 330 installed in a corresponding medial or lateral side of an ankle. Fig. 4 is an isometric view and Fig. 5 is a side view representing a corresponding lateral unicompartmental ankle prosthesis 430, 530 in an ankle. The structures of the medial unicompartmental ankle prosthesis 300 and the lateral unicompartmental ankle prosthesis 330 are mirror images of each other.

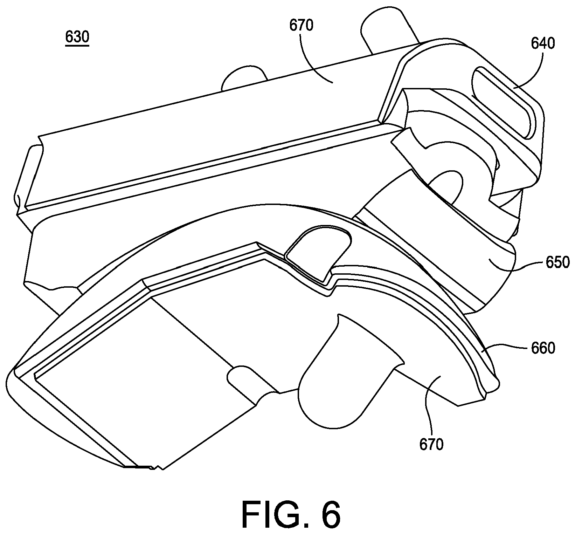

Fig. 6 is a bottom isometric view of a three-piece lateral unicompartmental ankle prosthesis 630 that includes a tibial component 640, a talar component 660, and a poly component 650. Optionally, as shown, the bone contacting surfaces of the unicompartmental ankle prosthesis 630 can be coated with a bioactive coating 670 to enhance bone growth and adhesion to the components while minimizing infection. That is, the top and side surfaces of the tibial component 640 and the bottom surface of the talar component 660 can be coated with the bioactive coating 670. The bioactive coating 670 can include beads, a plasma spray, a lattice foam structure, etc. This coating can be around 1mm thick and located where there is a bone to implant interface.

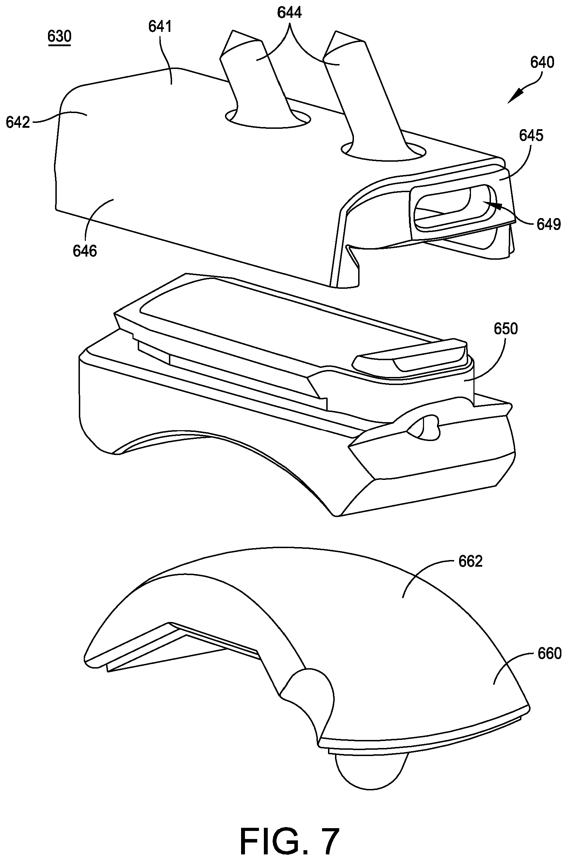

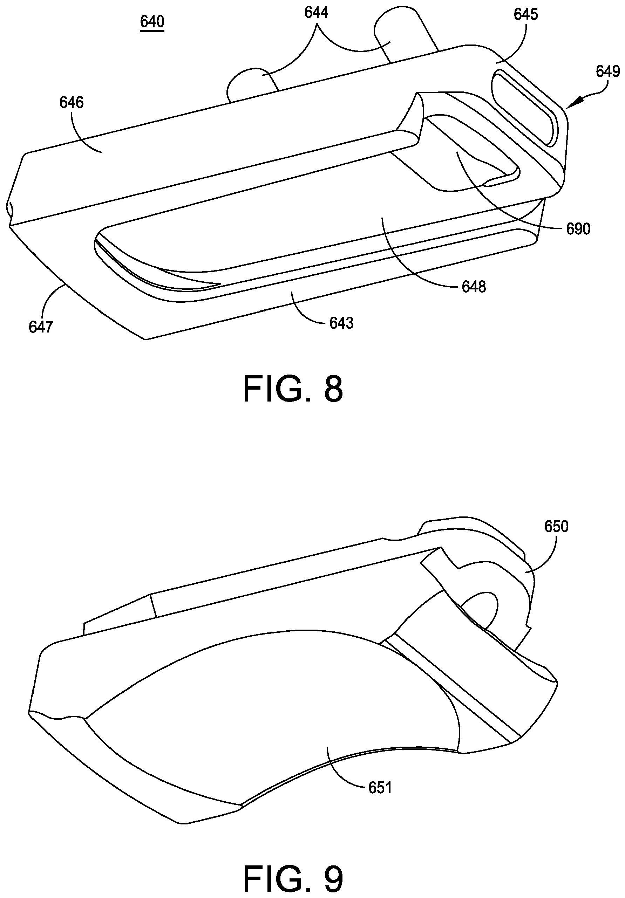

Fig. 7 is an exploded view of the lateral unicompartmental ankle prosthesis 630 of Fig. 6. Fig. 8 is an underside view of the tibial component 640 shown without the bioactive coating 670. As shown in Figs. 7 and 8, the tibial component 640 can have a substantially rectangular cuboid body 642 with one or more pegs 644 protruding out of a superior side 641 of the body 642. The pegs 644 are configured to be received in holes formed in the tibia. The tibial component 640 can also include two opposed shoulders 646 that are curved or chamfered relative to the superior side 641 of the body 642. A recessed area or recess 648 extends posteriorly from an anterior face 645 to an insertion end (or posterior end) 647 along the inferior side 643 of the tibial component 640. The recess 648 can be configured to receive sides and a curved edge of a tibial tray impaction insert tool as a means for holding the tibial component 640 in place during insertion. Additionally, the sides of the recess 648 can be undercut and angled to receive and engage with a dovetail feature of the poly component 650.

Bone cement (not shown) can be applied to the superior 641, medial, and lateral sides of the body 642 while a face of the anterior side 645 and inferior side 643 remains free of any cement.

As shown, the tibial component 640 can also include an opening 649 in the anterior face 645 to a cavity 690 that joins with the recess 648. The opening 649 can be used as an interface for a surgical assembly tool and to view a top of the poly component 650 to verify correct installation and alignment.

The tibial component 640 can be made of a cobalt chrome alloy, stainless steel, titanium, titanium alloy, or any other suitable material. Optionally, the tibial component 640 can be made using an additive manufacturing process such as 3-D printing. Optionally, the tibial component 640 can be made to fit an individual patient.

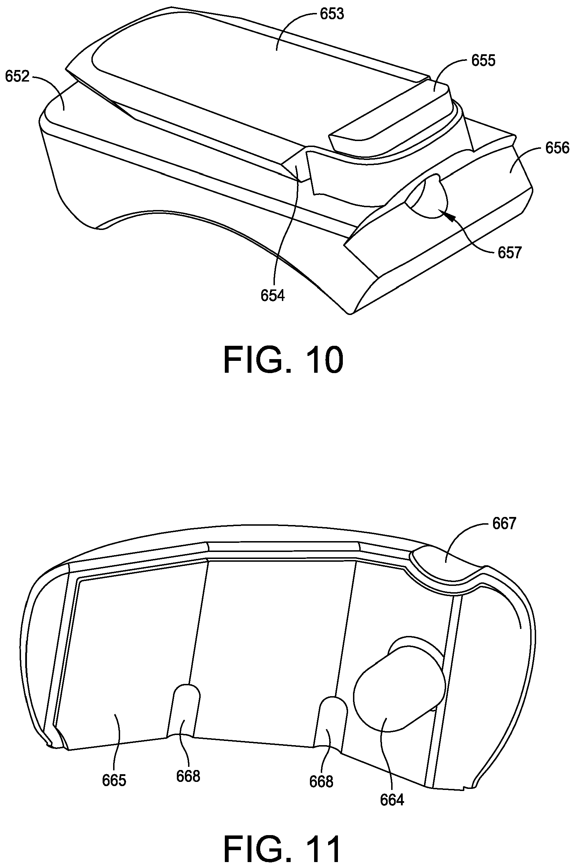

Fig. 9 is an underside view and Fig. 10 is a top side view of the poly component 650. As shown in Figs. 9 and 10, the poly component 650 can be substantially cubic with substantially flat sides and geometric features to engage and interface with surgical tooling, the tibial component 640, and the talar component 660. Fig. 9 shows that the inferior surface of the poly component 650 can include a recessed or concave surface 651 that defines an articulating surface to mate with a corresponding convex articulating surface of the talar component 660.

The superior side of the poly component 650 can include stepped surfaces that include a lower flat surface 652 that defines a substantially U-shape. When installed, the lower flat surface 652 will be adjacent to the inferior side 643 of the tibial component 640. Fig. 10 shows a protruding portion protruding from in between the legs of the U-shaped flat surface 652. A flat superior surface 653 defines most of the next stepped surface of the protruding portion. A U-shaped edge 654 of the protruding portion can be angled or chamfered to define a dovetail feature to fit within the corresponding undercut dovetail feature of the tibial component 640. A tab 655 with a flat top surface can protrude from the superior surface 653 and be configured to fit with the cavity 690 of the tibial component 640 and assist in locking together the poly component 650 and the tibial component 640 when the poly component 650 is installed.

The poly component 650 can also include an angled anterior notch 656 and a counterbore 657 on the anterior side both of which can be used to interface with tooling during the installation process. The poly component 650 can be made from or coated with a material that minimizes friction at the articular surface where the poly component 650 contacts the talar component 660. For example, the poly component 650 can be made from high-density polyethylene, polypropylene, or any other suitable material.

Fig. 11 is an underside view of the talar component 660 shown without the bioactive coating 670. As shown in Figs. 7 and 11, the tibial component 640 can have a substantially curved or crescent shape to match the geometry of the talar dome of the talus bone.

Fig. 7 shows that the talar component 660 includes a single continuous dome-shaped superior surface 662 that can be smooth or polished to facilitate articulation with the poly component 650. Talar component 660 is connected to the talus bone with one or more talar anterior pegs 664 that are located within holes provided in the talus. As shown in Fig. 11, the talar component 660 can include a notch 667 defined in at least one side surface that extends between superior surface 662 and anterior surface 665. Notch 667 may be defined by an inwardly curved, e.g. semi-cylindrical, depression in the selected side surface, and is sized and oriented to provide an interface or pilot for engagement with a portion of a surgical assembly tool thereby allowing accurate alignment of the prosthesis by the surgeon during installation. Also, as shown in Fig. 11, the anterior surface 665 of the talar component 660 defines one or more grooves 668. Grooves 668 may be defined by an inwardly curved, e.g. semi-cylindrical, depression in anterior surface 665, and are often oriented transversely on anterior surface 665. Grooves 668 provide added surface area to increase advantageously bone adhesion with the patient's talus. Other orientations of grooves 668, e.g., longitudinal or angled relative to a longitudinal or transverse axis of talar component 660, may be defined on anterior surface 665 to provide improved adhesion.

Optionally, bone cement can be applied to portions of the talar component 660 to help secure the talar component 660 to the talus. Also, a talar implant impactor tool can be used to complete seating of the talar component 664 to the talus.

The talar component 660 can be made of a cobalt chrome alloy, stainless steel, titanium, titanium alloy, or any other suitable material. Optionally, the talar component 660 can be made using an additive manufacturing process such as 3-D printing. Optionally, the talar component 660 can be made to fit an individual patient.

Upon completion of a partial ankle replacement procedure, the tibial component 640 is connected to the tibia, with pegs 644 located within peg holes in the resected tibia. The talar component 660 is connected to the talus, with talar dome anterior pegs 664 located within holes in the resected talus. The poly component 650 is inserted and located between the tibial component 640 and the talar component 660.

The medial unicompartmental ankle prosthesis 300 shown in Fig. 3 can include similar tibial, poly, and talar components with the same features as those described with respect to the lateral unicompartmental ankle prosthesis 630. For example, the tibial component 640 and the poly component 650 of the lateral unicompartmental ankle prosthesis 630 can be interchangeable with the tibial component and the poly component, respectively, of the medial unicompartmental ankle prosthesis 300. Additionally, the talar component of the medial unicompartmental ankle prosthesis 300 can be a mirrored copy of the talar component 660 of the lateral unicompartmental ankle prosthesis 630 in that the placement of the notch 667 and grooves 668 to interface with assembly tools are on different sides of the talar component.

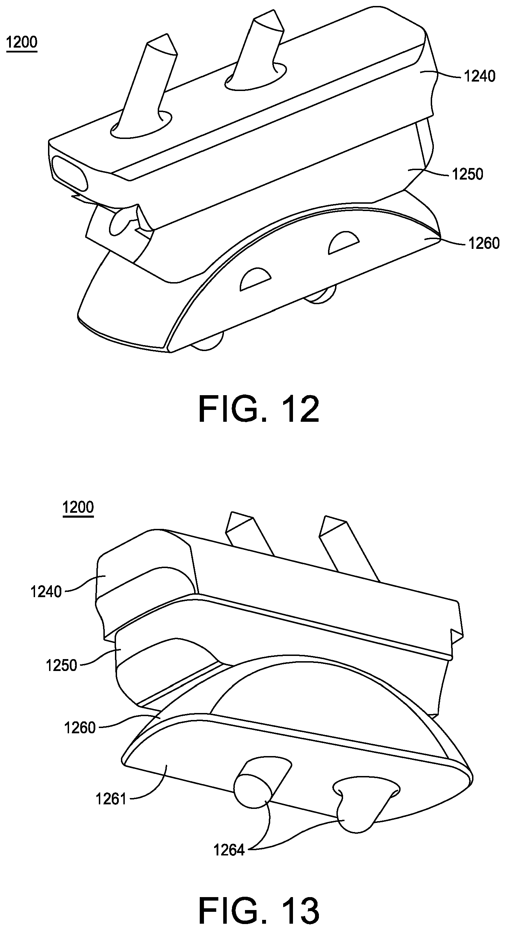

In another embodiment, a medial unicompartmental ankle prosthesis 1200 can include three components similar to that shown in Figs. 12 and 13 and previously described. The medial unicompartmental ankle prosthesis 1200 can include a tibial component 1240, a poly component 1250, and a talar component 1260. While the tibial component 1240 and the poly component 1250 can include the same features or be interchangeable with the tibial component 640 and the poly component 650 described above, the talar component 1260 is different than the talar component 660.

Figs. 12 and 13 show that the anterior surface 1261 of the talar component 1260 is flat. This can offer a surgical option to best fit a patient or in situations were more of the talus needs to be removed. Thus, the shape of the talar component 1260 is substantially hemispherical. The talar component 1260 is connected to the talus bone with one or more talar anterior pegs 1264 that are located within holes provided in the talus.

Although Fig. 3 shows medial and lateral unicompartmental ankle prostheses 300, 330 for a patient's right foot, the location for the two prostheses can also be switched on a patient's left foot. For example, the medial unicompartmental ankle prosthesis 300 for a right foot can be used as a lateral unicompartmental ankle prosthesis for a left foot. Likewise, the lateral unicompartmental ankle prosthesis 330 for a right foot can be used as a medial unicompartmental ankle prosthesis for a left foot. The difference between the medial and lateral unicompartmental ankle prostheses 300, 330 primarily being the mirrored location of the tooling interface features of the talar component 1260.

It should be understood that the foregoing description is only illustrative of the present invention. Various alternatives and modifications can be devised by those skilled in the art without departing from the present invention. Accordingly, the present invention is intended to embrace all such alternatives, modifications, and variances that fall within the scope of the appended claims.

## Claims

1. A partial ankle prosthesis (630) comprising:
a tibial component (640) including a superior surface (641) configured to be attached to a lower end of a patient's tibia;
a poly component (650) that is fixedly attached to the tibial component (640) and includes a concave inferior surface (651) of an anti-friction material; and
a talar component (630) including an inferior surface (665) configured to be attached to a patient's talus and a single convex superior surface (662) configured to mate with the inferior surface (651) of the poly component (650); **characterised in that**
the partial ankle prosthesis (630) is configured to be implanted in a single compartment of a patient's ankle, and is a lateral or medial unicompartmental ankle prosthesis.

2. The partial ankle prosthesis (630) of claim 1, wherein the superior surface (641) of the tibial component (640) includes a peg configured to fit into a hole in the patient's tibia.

3. The partial ankle prosthesis (630) of any of the preceding claims, wherein the anterior surface of the talar component (630) includes a peg configured to fit into a hole in the patient's talus.

4. The partial ankle prosthesis (630) of any of the preceding claims, wherein the superior surface (662) of the talar component (630) has only one continuous concave curvature both longitudinally between opposite anterior and posterior ends and laterally between opposite medial and lateral sides.

5. The partial ankle prosthesis (630) of any of the preceding claims, further comprising a bioactive coating (670) on a surface of the tibial component (640) and/or the talar component (630) that interfaces with a patient's bone.

6. The partial ankle prosthesis (630) of any of the preceding claims, wherein an inferior surface (665) of the talar component (630) is curved and configured to attach to a talar dome of the talus, or wherein an inferior surface (665) of the talar component (630) is substantially flat.

7. The partial ankle prosthesis (630) of any of the preceding claims, wherein the talar component (630) defines a notch arranged so as to interface with a surgical assembly tool.

8. The partial ankle prosthesis (630) of claim 1,
wherein the superior surface (641) of the tibial component has an outwardly projecting peg configured to be received within a corresponding passageway formed in a portion of a distal tibia; and
wherein the talar component (630) includes an inferior surface (665) having an outwardly projecting peg configured to be received within a corresponding passageway formed in a portion of a talus.

9. The partial ankle prosthesis (630) of claim 8, wherein the anterior surface of the talar component (630) defines at least groove so as to provide additional surface area and thereby increase bone adhesion, and/or wherein the superior surface (662) of the talar component (630) has only one continuous concave curvature both longitudinally between opposite anterior and posterior ends and laterally between opposite medial and lateral sides.

10. The partial ankle prosthesis (630) of any of claims 8-9, further comprising a bioactive coating on a surface of the tibial component (640) and/or the talar component (630) that interfaces with a patient's bone.

11. The partial ankle prosthesis (630) of any of claims 8-10, wherein an inferior surface (665) of the talar component (630) is curved and configured to attach to a talar dome of the talus, or wherein an inferior surface (665) of the talar component (630) is substantially flat.

12. The partial ankle prosthesis (630) of any of claims 8-11, wherein the talar component (630) defines a notch arranged so as to interface with a surgical assembly tool.

13. The partial ankle prosthesis (630) of claim 1,
wherein the talar component (630) includes a single convex superior surface (662) having one continuous concave curvature both longitudinally between anterior and posterior ends and laterally between medial and lateral sides, with a notch defined in at least one of the medial and lateral sides so as to interface with a surgical assembly tool.

14. The partial ankle prosthesis (630) of claim 13, wherein the superior surface (641) of the tibial component (640) includes a peg configured to fit into a hole in the patient's tibia, and/or wherein the anterior surface of the talar component (630) includes a peg configured to fit into a hole in the patient's talus.

15. The partial ankle prosthesis (630) of any of claims 13-14, further comprising a bioactive coating on a surface of the tibial component (640) and/or the talar component (630) that interfaces with a patient's bone, and/or wherein an inferior surface (665) of the talar component (630) is curved and configured to attach to a talar dome of the talus.

## Patentansprüche

1. Partielle Sprunggelenkprothese (630), umfassend:
eine Tibiakomponente (640), die eine obere Oberfläche (641umfasst, die konfiguriert ist, um an einem unteren Ende einer Tibia eines Patienten befestigt zu werden;
eine Polykomponente (650), die an der Tibiakomponente (640) fest befestigt ist und eine konkave untere Oberfläche (651) aus einem reibungsmindernden Material umfasst; und
eine Taluskomponente (630), die eine untere Oberfläche (665), die konfiguriert ist, um an einem Talus eines Patienten befestigt zu werden, und eine einzelne konvexe obere Oberfläche (662) umfasst, die konfiguriert ist, um mit der unteren Oberfläche (651) der Polykomponente (650) zusammenzupassen;
**dadurch gekennzeichnet, dass**
die partielle Sprunggelenkprothese (630) konfiguriert ist, um in einem einzelnen Kompartiment des Sprunggelenks eines Patienten implantiert zu werden, und eine laterale oder mediale unikompartimentelle Sprunggelenkprothese ist.

2. Partielle Sprunggelenkprothese (630) nach Anspruch 1, wobei die obere Oberfläche (641) der Tibiakomponente (640) einen Zapfen umfasst, der konfiguriert ist, um in ein Loch in der Tibia des Patienten zu passen.

3. Partielle Sprunggelenkprothese (630) nach einem der vorstehenden Ansprüche, wobei die vordere Oberfläche der Taluskomponente (630) einen Zapfen umfasst, der konfiguriert ist, um in ein Loch in dem Talus des Patienten zu passen.

4. Partielle Sprunggelenkprothese (630) nach einem der vorstehenden Ansprüche, wobei die obere Oberfläche (662) der Taluskomponente (630) sowohl in Längsrichtung zwischen gegenüberliegenden vorderen und hinteren Enden als auch in Querrichtung zwischen gegenüberliegenden medialen und queren Seiten nur eine durchgehende konkave Krümmung aufweist.

5. Partielle Sprunggelenkprothese (630) nach einem der vorstehenden Ansprüche, ferner umfassend eine bioaktive Beschichtung (670) auf einer Oberfläche der Tibiakomponente (640) und/oder der Taluskomponente (630), die mit einem Knochen eines Patienten in Verbindung steht.

6. Partielle Sprunggelenkprothese (630) nach einem der vorstehenden Ansprüche, wobei eine untere Oberfläche (665) der Taluskomponente (630) gekrümmt und konfiguriert ist, um an einer Talusrolle des Talus befestigt zu werden, oder wobei eine untere Oberfläche (665) der Taluskomponente (630) im Wesentlichen flach ist.

7. Partielle Sprunggelenkprothese (630) nach einem der vorstehenden Ansprüche, wobei die Taluskomponente (630) eine Kerbe definiert, die angeordnet ist, um mit einem chirurgischen Montagewerkzeug in Verbindung zu stehen.

8. Partielle Sprunggelenkprothese (630) nach Anspruch 1,
wobei die obere Oberfläche (641) der Tibiakomponente einen nach außen vorstehenden Zapfen aufweist, der konfiguriert ist, um innerhalb eines entsprechenden Durchgangs aufgenommen zu werden, der in einem Abschnitt einer distalen Tibia ausgebildet ist; und
wobei die Taluskomponente (630) eine untere Oberfläche (665) umfasst, die einen nach außen vorstehenden Zapfen aufweist, der konfiguriert ist, um innerhalb eines entsprechenden Durchgangs aufgenommen zu werden, der in einem Abschnitt eines Talus ausgebildet ist.

9. Partielle Sprunggelenkprothese (630) nach Anspruch 8, wobei die vordere Oberfläche der Taluskomponente (630) mindestens eine Nut definiert, um einen zusätzlichen Oberflächenbereich bereitzustellen und dadurch die Knochenadhäsion zu erhöhen, und/oder wobei die obere Oberfläche (662) der Taluskomponente (630) nur eine kontinuierliche konkave Krümmung sowohl in Längsrichtung zwischen gegenüberliegenden vorderen und hinteren Enden als auch in Querrichtung zwischen gegenüberliegenden medialen und queren Seiten aufweist.

10. Partielle Sprunggelenkprothese (630) nach einem der Ansprüche 8 bis 9, ferner umfassend eine bioaktive Beschichtung auf einer Oberfläche der Tibiakomponente (640) und/oder der Taluskomponente (630), die mit einem Knochen eines Patienten in Verbindung steht.

11. Partielle Sprunggelenkprothese (630) nach einem der Ansprüche 8 bis 10, wobei eine untere Oberfläche (665) der Taluskomponente (630) gekrümmt und konfiguriert ist, um an einer Talusrolle des Talus befestigt zu werden, oder wobei eine untere Oberfläche (665) der Taluskomponente (630) im Wesentlichen flach ist.

12. Partielle Sprunggelenkprothese (630) nach einem der Ansprüche 8 bis 11, wobei die Taluskomponente (630) eine Kerbe definiert, die angeordnet ist, um mit einem chirurgischen Montagewerkzeug in Verbindung zu stehen.

13. Partielle Sprunggelenkprothese (630) nach Anspruch 1,
wobei die Taluskomponente (630) eine einzelne konvexe obere Oberfläche (662) umfasst, die eine durchgehende konkave Krümmung sowohl in Längsrichtung zwischen vorderen und hinteren Enden als auch in Querrichtung zwischen medialen und queren Seiten aufweist, wobei eine Kerbe in mindestens einer der medialen und der queren Seite definiert ist, um mit einem chirurgischen Montagewerkzeug in Verbindung zu stehen.

14. Partielle Sprunggelenkprothese (630) nach Anspruch 13, wobei die obere Oberfläche (641) der Tibiakomponente (640) einen Zapfen umfasst, der konfiguriert ist, um in ein Loch in der Tibia des Patienten zu passen, und/oder wobei die vordere Oberfläche der Taluskomponente (630) einen Zapfen umfasst, der konfiguriert ist, um in ein Loch in dem Talus des Patienten zu passen.

15. Partielle Sprunggelenkprothese (630) nach einem der Ansprüche 13 bis 14, ferner umfassend eine bioaktive Beschichtung auf einer Oberfläche der Tibiakomponente (640) und/oder der Taluskomponente (630), die mit einem Knochen eines Patienten in Verbindung steht, und/oder wobei eine untere Oberfläche (665) der Taluskomponente (630) gekrümmt und konfiguriert ist, um an einer Talusrolle des Talus befestigt zu werden.

## Revendications

1. Prothèse de cheville partielle (630) comprenant :
un composant tibial (640) comprenant une surface supérieure (641) configurée pour être fixée à une extrémité inférieure du tibia d'un patient ;
un composant en polymère (650) qui est fixé, de manière fixe, au composant tibial (640) et comprend une surface inférieure concave (651) d'un matériau antifriction ; et
un composant astragalien (630) comprenant une surface inférieure (665) configurée pour être fixée à l'astragale d'un patient et une surface supérieure convexe (662) unique configurée pour se coupler avec la surface inférieure (651) du composant en polymère (650) ; **caractérisée en ce que** :
la prothèse de cheville partielle (630) est configurée pour être implantée dans un compartiment unique de la cheville d'un patient, et est une prothèse de cheville unicompartimentale latérale ou médiale.

2. Prothèse de cheville partielle (630) selon la revendication 1, dans laquelle la surface supérieure (641) du composant tibial (640) comprend une cheville configurée pour s'adapter dans un trou dans le tibia du patient.

3. Prothèse de cheville partielle (630) selon l'une quelconque des revendications précédentes, dans laquelle la surface antérieure du composant astragalien (630) comprend une cheville configurée pour s'adapter dans un trou dans l'astragale du patient.

4. Prothèse de cheville partielle (630) selon l'une quelconque des revendications précédentes, dans laquelle la surface supérieure (662) du composant astragalien (630) n'a qu'une courbure concave continue à la fois longitudinalement entre les extrémités antérieure et postérieure opposées et latéralement entre les côtés médial et latéral opposés.

5. Prothèse de cheville partielle (630) selon l'une quelconque des revendications précédentes, comprenant en outre un revêtement bioactif (670) sur une surface du composant tibial (640) et/ou le composant astragalien (630) qui s'interface avec l'os d'un patient.

6. Prothèse de cheville partielle (630) selon l'une quelconque des revendications précédentes, dans laquelle une surface inférieure (665) du composant astragalien (630) est courbée et configurée pour se fixer à un dôme astragalien de l'astragale, ou bien dans laquelle une surface inférieure (665) du composant astragalien (630) est sensiblement plate.

7. Prothèse de cheville partielle (630) selon l'une quelconque des revendications précédentes, dans laquelle le composant astragalien (630) définit une encoche agencée pour s'interfacer avec un outil d'assemblage chirurgical.

8. Prothèse de cheville partielle (630) selon la revendication 1,
dans laquelle la surface supérieure (641) du composant tibial a une cheville en saillie vers l'extérieur configurée pour être reçue dans une voie de passage correspondante formée dans une partie d'un tibia distal ; et
dans laquelle le composant astragalien (630) comprend une surface inférieure (665) ayant une cheville en saillie vers l'extérieur configurée pour être reçue dans une voie de passage correspondante formée dans une partie d'un astragale.

9. Prothèse de cheville partielle (630) selon la revendication 8, dans laquelle la surface antérieure du composant astragalien (630) définit au moins une rainure afin de fournir une surface supplémentaire et ainsi accroître l'adhérence osseuse et/ou dans laquelle la surface supérieure (662) du composant astragalien (630) n'a qu'une courbure concave continue à la fois longitudinalement entre les extrémités antérieure et postérieure opposées et latéralement entre les côtés médial et latéral opposés.

10. Prothèse de cheville partielle (630) selon l'une quelconque des revendications 8 à 9, comprenant en outre un revêtement bioactif sur une surface du composant tibial (640) et/ou du composant astragalien (630) qui s'interface avec l'os d'un patient.

11. Prothèse de cheville partielle (630) selon l'une quelconque des revendications 8 à 10, dans laquelle une surface inférieure (665) du composant astragalien (630) est courbée et configurée pour se fixer à un dôme astragalien de l'astragale, ou bien dans laquelle une surface inférieure (665) du composant astragalien (630) est sensiblement plate.

12. Prothèse de cheville partielle (630) selon l'une quelconque des revendications 8 à 11, dans laquelle le composant astragalien (630) définit une encoche agencée pour s'interfacer avec un outil d'assemblage chirurgical.

13. Prothèse de cheville partielle (630) selon la revendication 1,
dans laquelle le composant astragalien (630) comprend une surface supérieure convexe (662) unique ayant une courbure concave continue à la fois longitudinalement entre les extrémités antérieure et postérieure et latéralement entre les côtés médial et latéral, avec une encoche définie dans au moins l'un des côtés médial et latéral afin de s'interfacer avec un outil d'assemblage chirurgical.

14. Prothèse de cheville partielle (630) selon la revendication 13, dans laquelle la surface supérieure (641) du composant tibial (640) comprend une cheville configurée pour s'adapter dans un trou dans le tibia du patient, et/ou dans laquelle la surface antérieure du composant astragalien (630) comprend une cheville configurée pour s'adapter dans un trou dans l'astragale du patient.

15. Prothèse de cheville partielle (630) selon l'une quelconque des revendications 13 à 14, comprenant en outre un revêtement bioactif sur une surface du composant tibial (640) et/ou du composant astragalien (630) qui s'interface avec l'os d'un patient, et/ou dans laquelle une surface inférieure (665) du composant astragalien (630) est courbée et configurée pour se fixer à un dôme astragalien de l'astragale.
